## Europäisches Patentamt
## European Patent Office
## Office européen des brevets

(11) Veröffentlichungsnummer: **0 196 353**
**A2**

# (12) EUROPÄISCHE PATENTANMELDUNG

(21) Anmeldenummer: 85104138.4

(51) Int. Cl.⁴: **A 61 B 17/22**

(22) Anmeldetag: 04.04.85

(43) Veröffentlichungstag der Anmeldung:
08.10.86 Patentblatt 86/41

(84) Benannte Vertragsstaaten:
AT BE CH DE FR GB IT LI NL SE

(71) Anmelder: DORNIER SYSTEM GmbH
Postfach 1360
D-7990 Friedrichshafen(DE)

(72) Erfinder: Wess, Othmar, Dr.
Hersbergweg 6
D-7997 Immenstaad(DE)

(72) Erfinder: Marlinghaus, Ernst
Kapitän-Wagner-Strasse 34
D-7990 Friedrichshafen(DE)

(72) Erfinder: Hepp, Wolfgang, Dr.
Hardtstrasse 6
D-7997 Immenstaad(DE)

(72) Erfinder: Heine, Gerold, Dr.
Reismühlenweg 7
D-7772 Uhldingen-Mühlhofen 1(DE)

(74) Vertreter: Landsmann, Ralf, Dipl.-Ing.
Kleeweg 3
D-7990 Friedrichshafen 1(DE)

(54) Vorrichtung zur Vermeidung oder Minderung von Schmerzen bei der extracorporalen Lithotripsie.

(57) Bei der extracorporalen Lithotropsie mittels durch eine Funkenstrecke erzeugter Stosswellen wird die nachfolgende technisch nicht verweidbare Druckwelle durch eine als Filter wirkende Kugelkalotte am Stosswellenerzeuger herausgefiltert. Dadurch werden Schmerzwirkungen beim Patienten vermieden oder vermindert.

Fig. 1

Vorrrichtung zu Vermeidung oder Minderung von Schmerzen bei der extracorporalen Lithotripsie

Die Erfindung betrifft eine Vorrichtung zur Vermeidung oder Minderung von Schmerzen bei der Lithotripsie von nicht-anästhesierten Patienten mittels Stosswellen und ist sowohl dann anwendbar, wenn sich Patient und Stosswellenquelle in einem Wasserbad befinden oder aber die Behandlung wannenfrei erfolgt.

Die Stosswellenlithotripsie an nicht anästhesierten Patienten ist mit einer erheblichen Schmerzwirkung verbunden. Dies ist wie Schlierenuntersuchungen und Druckmessungen zeigen darauf zurückzuführen, dass auf die eigentliche Stosswelle eine langsame Druckwelle folgt, die aufgrund ihres zeitlichen Ablaufs (einige Millisekunden ) die Schmerzwirkung auslöst und selbst nicht zur Zertrümmerung des Konkrements beiträgt, während die sehr viel schnellere Stosswelle ( ca. 0.5 Mikrosekunden ) , die das Konkrement zertrümmert, kaum eine Schmerzwirkung auslöst. Die Erzeugung von Stosswellen mit Unterwasserfunkenentladung oder Unterwasserdrahtentladung ist ursächlich mit der Bildung von niederfrequenten Druckanteilen verbunden, da die Expansion des Funkenplasmas zu einer grossvolumigen Verdrängung des umgebenden Wassers führt. Die daraus resultierenden niederfrequenten Druckpulse und Schwallströmungen treten ungehindert aus der Ellipspoidöffnung auf den Patienten und führen damit zu einer Schlagbelastung.

Aus der Deutschen Offenlegungsschrift 29 13251 ist es bekannt, den

Stosswellenerzeuger mittels einer ebenen metallischen Membran zu verschliessen, wobei sich zeigte, dass die Druckwelle die ebene metallische Membran ausbeulte und beim zurückfedern Gewebe einklemmen konnte. Abhilfe für das Einklemmen von Gewebe schaffte ein Wassersack zwischen Membran und Körper des Patienten, ohne dass dadurch die Schmerzwirkung der Druckwelle verhindert oder vermieden werden konnte.

Aus der Deutschen Patentschrift 23 51247 ist es bekannt, den Stosswellenezeuger mit einer elastichen Membran zu verschliessen, die sich luftspaltlos an den Körper des Patienten anlegt und damit der Kontur des Körpers folgend eine Wölbung bildet. Diese Membran lässt die Stosswelle und auch die nachfolgende Druckwelle ungehindert passieren, sodass eine Reduzierung der Schmerzbelastung nicht erfolgt.

Der Erfindung liegt die Aufgabe zugrunde, eine Vorrichtung zu schaffen, die es bei der berührungslosen Zertrümmerung von Konkrementen mittels Stosswellen möglich macht, die auf die Stosswelle folgende Druckwelle herauszufiltern und dadurch die Schmerzbelastung beim nicht anästhesierten Patienten zu vermeiden oder zu vermindern.

Diese Aufgabe wird erfindungsgemäss durch die Vorrichtung gemäss den Patentansprüchen gelöst, wobei vorteilhafte Ausgesaltungen der Erfindung in den Unteransprüchen angegeben sind

Mit Hilfe des Gegenstands der Erfindung kann ohne Beeinträchtigung

der Zertrümmerungswirkung der Stosswelle die Schmerzempfindung dadurch weitgehend verhindert werden, dass die Stosswelle von der nachfolgenden Druckwelle getrennt wird, sodass allein die Stosswelle auf den Patienten einwirkt. Eine Trennung der beiden Anteile ist dadurch möglich, dass eine formstabile stosswellendurchlässige Kugelkalotte zwischen dem Ort der Stosswellenerzeugung und dem Patienten eingefügt wird. Die Kalotte verhindert, dass sich die langsame Druckwelle auf den Patienten überträgt.

Die Erfindung wird nachfolgend an Hand von Figuren näher erläutert. Es zeigen:

Fig. 1 ein schematisches Druck-Zeit-Profil für eine Stosswelle mit nachfolgender Druckwelle,

Fig. 2 eine Einrichtung zur extracorporalen Lithotripsie mit der erfindungsgemässen Vorrichtung, wobei sich der Patient in einem Wasserbad befindet,

Fig. 3 eine Einrichtung zur extacorporalen Lithotripsie mit der erfindungsgemässen Vorrichtung, bei wannenfreier Behandlung des Patienten und

Fig. 4 - 6 Einzelheiten und Ausgestaltungen der erfindungsgemässen Vorrichtung.

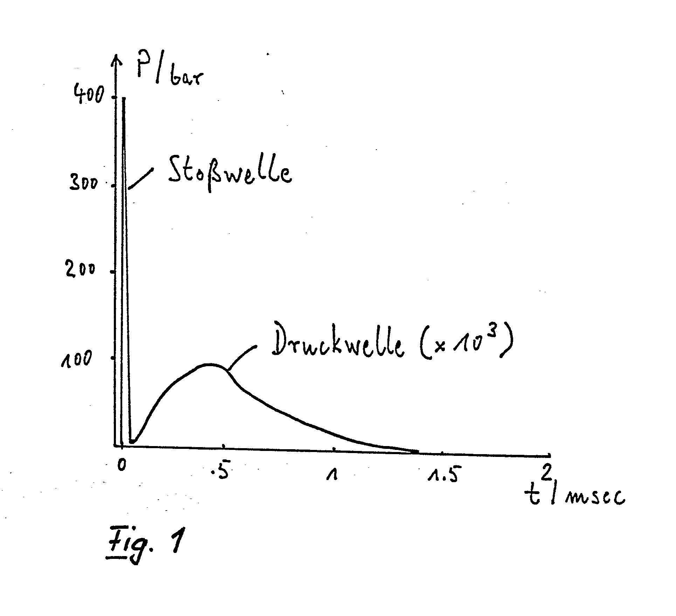

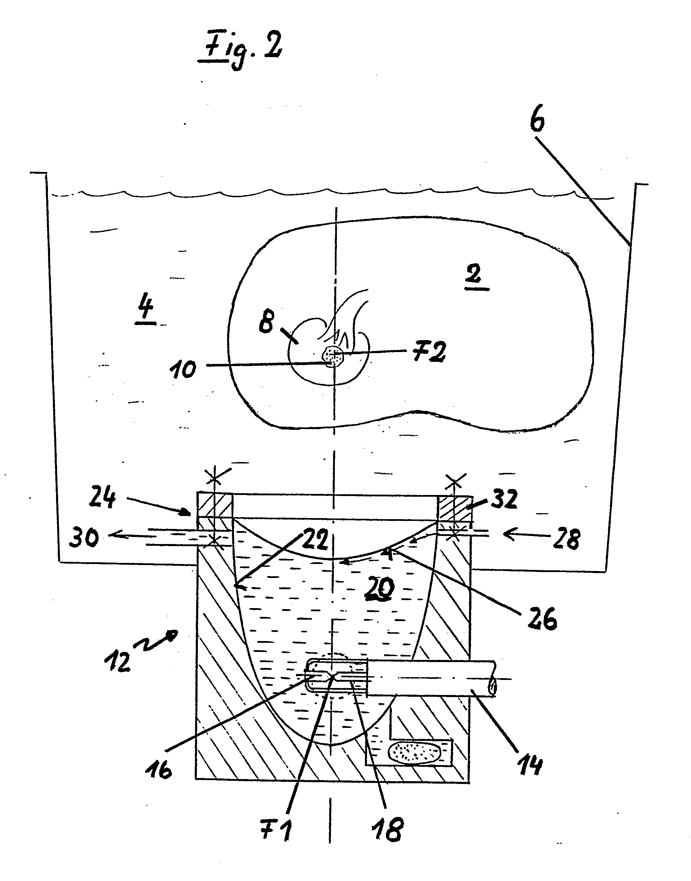

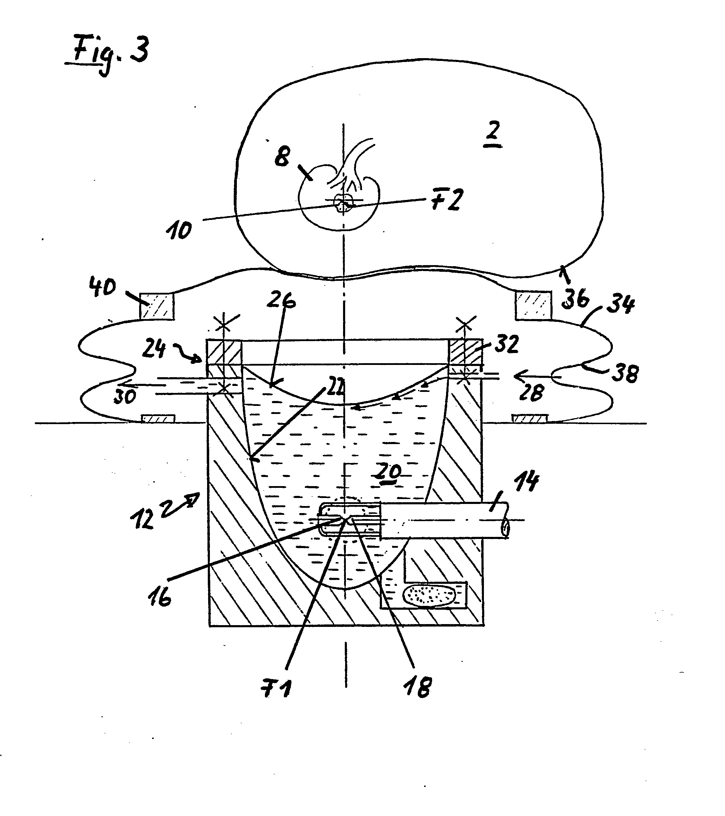

Fig. 1 zeigt das schematische Druck-Zeitprofil einer fokussierenden Unterwasserfunkenquelle wie sie beispielsweise von einer in Fig. 2 und 3 dagestellten Elektrode erzeugt wird. Die im linken Teil der Fig. gezeigte Stosswelle hat einen steilen Anstieg und einen steilen Abfall. Ihre Dauer beträgt ca. 0.5 Mikrosekunden, wobei ein hoher Druck von ca. 400 bar erreicht wird. Auf die Stosswelle folgt, ohne dass dies technisch zu verhindern ist, eine langsame Druckwelle, deren Dauer 1000-fach länger ist als die Stosswelle und deren Druckamplitude typisch zwischen 100 mbar und 1 bar liegt Die Zertrümmerung eines Konkrements wird ausschliesslich von der Stosswelle bewirkt, die nachfolgende Druckwelle nimmt an der Zertrümmerung nicht teil und verursacht Beim Patienten ein Schmerzgefühl.

Fig. 2 zeigt prinzipiell die extracorporale Lithotripsie an einem Patienten 2, der sich in einem Wasserbad 4 innerhalb einer Wanne 6 befindet Dabei wird die Niere 8 des Patienten 2, die einen Nierenstein 10 aufweist in den zweiten Fokuspunkt F2 bezüglich einer Fokussierungskammer 12 gefahren. Die Fokussierungskammer besteht aus einem Teil eines Rotationsellipsoids, wobei sich im Fokus F1 eine Funkenstrecke befindet, deren Elektrode 14 in der Figur dargestellt ist. Die Stosswelle und nachfolgende Druckwelle werden dadurch erzeugt, dass zwischen den Elektrodenspitzen 16, 18 ein Funke überspringt, wobei die Einrichtungen zur Erzeugung des Funkens in der Figur nicht dargestellt sind. Die Stosswelle wird in dem mit Wasser 20 gefüllten Ellipsoid 22 fokussiert und trifft auf den in F2 befindlichen Nierenstein.

Die Druckwelle wird nicht oder nur wenig fokussiert und breitet sich innerhalb des Ellipsoids 22 aus. In der Nähe des oberen Randes 24 des Ellipsoids befindet sich eine Kugelkalotte 26, die formstabil nach innen ( in das Ellipsoid 22 hinein ) gewölbt ist und aufgrund ihrer Wölbung, ihres Materials , ihrer Wandstärke und ihrer starren Einspannung im Rand 24 die Druckwelle nahezu vollständig herausfiltert, während die Stosswellenfront nahezu parallel zur Kalotte verläuft und dadurch keine Brechung erleidet. es ist nicht damit zu rechnen, dass sich Gasblasen an der Kalotte absetzen, da sie nach innen gewöbt ist. Dies kann zusätzlich dadurch verhindert werden, dass in Nähe der Kalotte 26 ein Wassereinlauf 28 und ein Wasserablauf 30 vorhanden sind, wodurch eine für die Entfernung von Luftblasen sehr wirksame Tangentialströmung sehr dicht an der Kalotte entsteht.

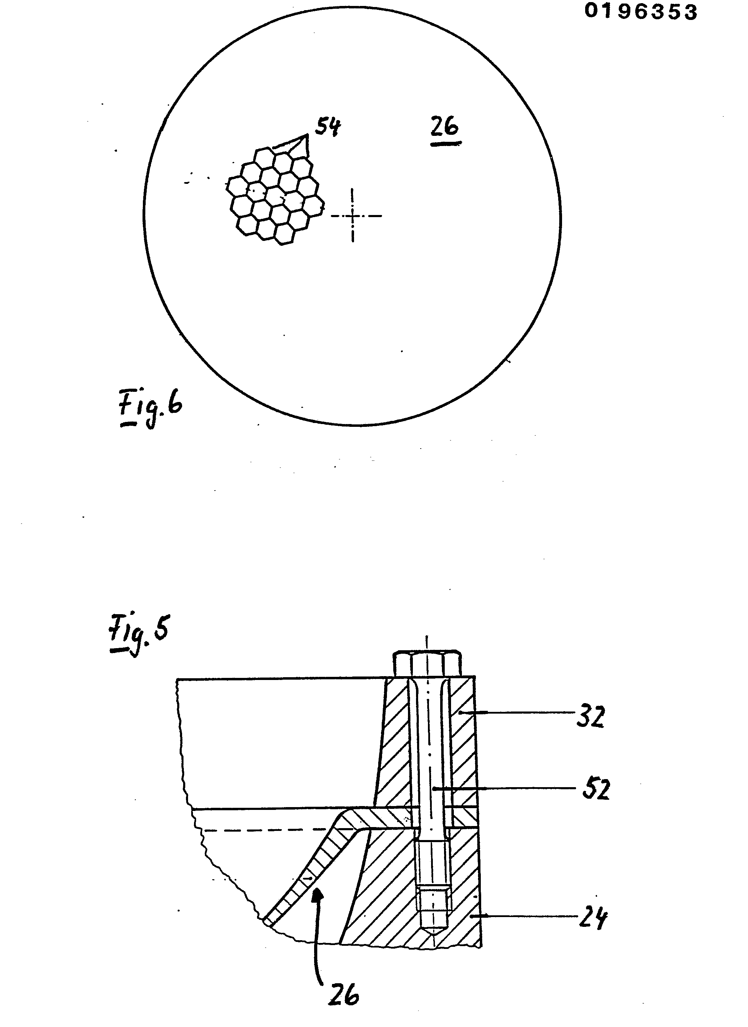

Die Kalotte 26 wird mit einem Druckring 32 an den Rand 24 der Fokussierungskammer 12 angepresst. Einzelheiten sind in Fig. 5 gezeigt.

Fig. 3 zeigt ein Ausführungsbeispiel für eine wannenfreie extracorporale Lithotripsie, wobei an Stelle der Wanne ein mit Wasser gefüllter Beutel 34 verwendet wird, der einerseits mit der Fokussierungskammer 12 verbunden ist und andererseits an der Körperoberfläche 36 des Patienten 2 anliegt. Der Beutel weist zur Anpassung an die Lage des Patienten einen Faltenbalg 38 und einen Ring 40 auf. Die in Figur 3 gezeigte Kalotte 26 entspricht der in Figur 2 gezeigten Kugelkalotte.

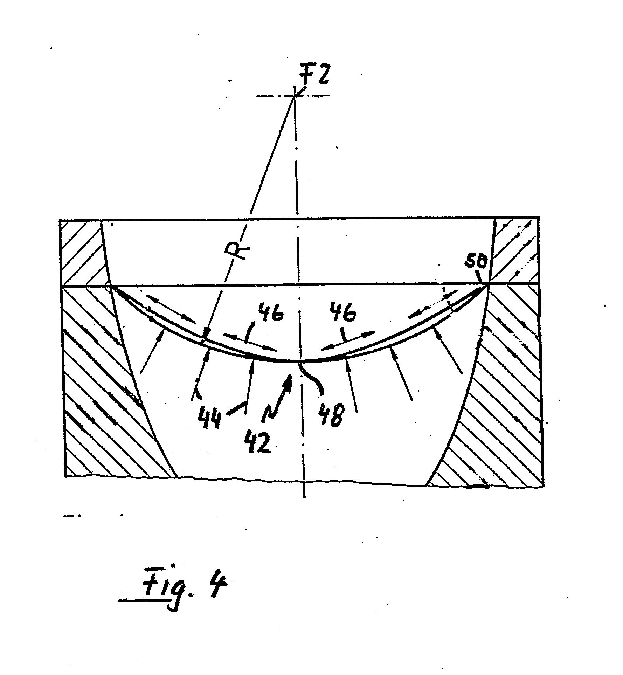

Einzelheiten der erfindungsgemässen Kugelkalotte sind in den Figuren 4 bis 6 gezeigt. Fig 4 zeigt eine Kugelkalotte 42, die im Querschnitt eine Gewöbescheitelkurve ist und die Eigenschaft hat, dass Druckbelastungen (angedeutet durch Pfeile 44 ) in reine Druckspannungen ( angedeutet durch Pfeile 46) umgewandelt werden.

Fig 4 zeigt auch, dass der Krümmungsmittelpunkt der kreisförmigen Kugelkalotte in vorteilhafter Weise der zweite Brennpunkt F2 sein kann. Ferner ist in Fig. 4 die Wandstärke der Kugelkalotte im Zentrum 48 und im Randbereich 50 geringer als in den übrigen Bereichen. Die Wandstärke der Kugelkalotte ist so zu wählen, dass sie kleiner als die Wellenlänge der Stosswelle ist. Bei Kugelkalotten aus Stahl hat sich eine Wandstärkendicke von 0.3 mm als geeignet erwiesen.

Fig 5 zeigt die Einspannung der Kugelkalotte im Rand 24 der Fokussierungskammer 12 , wobei die Kalotte mittels eines Druckrings 32 an der Fokussierungskammer befestigt wird. Zur sicheren Übertragung, Aufnahme und Abführung der Druckwellen wird der Druckring mit Dehnschrauben 52 befestigt, die die Eigenschaft haben, dass sie Zugbelastungen elastisch aufnehmen können. Ferner zeigt Fig. 5 einen Teil einer Kugelkalotte, bei dem die Wandstärke vom Rand aus kontinuierlich abnimmt.

Fig. 6 zeigt als Einzelheit eine Kugelkalotte 26 in Draufsicht, die mittels Prägen oder Drücken eine facettenartige Struktur aufweist. Die Facetten 54 können sechseckig sein oder andere geometrische Muster bilden. In die Kalotte können auch Sollbiegestellen eingebaut werden, um zu verhindern, dass plasstisch eingebeulte Bereiche auftreten. Als Material für die Kugelkalotten mit oder ohne Facettenstruktur kommen Werkstoffe mit hohem Elastizitätsmodul in Frage. Dies sind unter anderem legierte und unlegierte Stähle, Kupfer-Beryllium-Legierungen und Kunststoffe aus Kohlenstoffasern.

Erfindungswesentlich ist dass die Kugelkalotte während einer langen Standzeit formstabil bleibt und die geometrisch vogegebene Kalottenform beibehält, da sowohl Wölbung wie Material , Wandstärke und Einspannung für das Herausfiltern der Druckwelle verantwortlich sind.

Beispiele

| Werkstoff | E- Modul $10^3\,N/mm^2$ | Wandstärke mm |
|-----------|-------------------------|---------------|
| Stahl     | 190.....220             | 0.2.......0.5 |
| Cu-Be     | $\approx 125$           | 0.2.....0.5   |
| CFK       | $\approx 100$           | 0.2......0.5  |

1

Patentansprüche

1. Vorrichtung zur Vermeidung oder Minderung von Schmerzen bei der extracorporalen Lithotripsie von nicht-anästhesierten Patienten, wobei sich innerhalb eines Wasserbads eine rotationselliptische Fokussierungskammer mit Stosswellenquelle in ihrem Brennpunkt und der zu behandelnde Patient mit dem Konkrement im zweiten Brennpunkt befinden, dadurch gekennzeichnet, dass die Fokussierungskammer (12) mit einer formstabil gewölbten Kugelkalotte (26) verschlossen ist, deren Wandstärke wesentlich kleiner als die Wellenlänge der Stosswelle ist und deren Wölbung in das Ellipsoid (22) hineinragt.

2. Vorrichtung zur Vermeidung oder Minderung von Schmerzen bei der extracorporalen Lithotripsie von nicht anästhesiertten Patienten mittels einer wassergefüllten rotationselliptischen Fokussierungskammer, in deren einem Brennpunkt sich eine Stosswellenquelle befindet, wobei die Fokussierungskammer abgeschlossen ist und die Ankopplung an den Patienten mittels eines wassergefüllten Sacks oder Beutels erfolgt, dadurch gekennzeichnet, dass eine formstabil gewölbte Kugelkalotte (26) das Ellipsoid (22) abschliesst, deren Wandstärke wesentlich kleiner als die Wellenlänge der Stosswelle ist und die Kugelkalotte (26) in das Ellipsoid hineinragt.

3. Kugelkalotte nach Ansprüchen 1 oder 2, dadurch gekennzeichnet, dass ihr Querschnitt eine Gewölbescheitelkurve ist, in der Druckbelastungen reine Druckspannungen erzeugen.

4.Kugelkalotte nach Ansprüchen 1 oder 2, dadurch gekennzeichnet, dass der Krümmungsmittelpunkt der Kugelkalotte (26) der zweite Brennpunkt F2 bezogen auf das Ellipsoid (22) ist.

5. Kugelkalotte nach Ansprüchen 1 bis 4 , dadurch gekennzeichnet, dass der Rand der Kalotte im Rand des Ellipsoids mittels eines Rings (32) starr eingespannt ist und der Ring mit Dehnschrauben (52) am Ellipsoid (22) befestigt ist.

6. Kugelkalotte nach Ansprüchen 1 bis 5, dadurch gekennzeichnet, dass die Kalotte (26) aus einem Material mit hohem Elastizitätsmodul wie Stahl, CFK, Cu-Be-Legierung besteht.

7. Kugelkalotte nach Ansprüchen 1 bis 6, dadurch gekennzeichnet, dass zur völligen galvanischen Trennung des Elektrodenraums vom Patientenraum die Kugelkalotte (26) geerdet ist.

8. Kugelkalotte nach Ansprüchen 1 bis 7, dadurch gekennzeichnet, dass Versteifungen oder Sollbiegestellen vorhanden sind.

9. Kugelkalotte nach Ansprüchen 1 bis 8 , dadurch gekennzeichnet, dass die Kalotte (26) zumindest auf einer Seite eine facettenstruktur aufweist.

10. Kugelkalotte nach Ansprüchen 1 bis 9, dadurch gekennzeichnet, dass die Kalotte (26) in ihrem Zentrum dünner ist als in den übrigen Bereichen und ihre Wandstärke kontinuierlich zum Zentrum hin abnimmt.

11. Kugelkalotte nach Ansprüchen 1 bis 10, dadurch gekennzeichnet, dass die Kalotte (26) im Zentrum und in den Randbereichen dünner ist als in den übrigen Bereichen.

12. Vorrichtung zur Minderung der Druckwelle am Entstehungsort nach den Ansprüchen 1 und 2, dadurch gekennzeichnet, dass in Nähe der Funkenstrecke (14) sich elastisch verformbare Elemente in Ausnehmungen in der Wand der Fokussierungskammer befinden.

13. Vorrichtung nach Ansprüchen 1 - 2, dadurch gekennzeichnet, dass ein Wasserzulauf (28) und ein Wasserablauf (30) in Nähe der Kalotte (26) im Ellipsoid (22) vorgesehen sind.

02.04.85/PaL

115

Fig. 1

0196353

Fig. 2

## Fig. 3

Fig. 4

515

0196353

**Fig.6**

26

54

**Fig.5**

32

52

24

26